# EUROPÄISCHE PATENTSCHRIFT

(11) **EP 3 714 934 B1**
(45) Veröffentlichungstag und Bekanntmachung des Hinweises auf die Patenterteilung: **15.05.2024**
(21) Anmeldenummer: 20162031.7
(22) Anmeldetag: 10.03.2020
(51) Int. Cl.: A61M 39/20

(54) **MEDIZINISCHER FLUIDKONNEKTOR**
MEDICAL FLUID CONNECTOR
CONNECTEUR DE FLUIDE MÉDICAL

(30) Priorität: 27.03.2019 DE 102019204211
(43) Veröffentlichungstag der Anmeldung: 30.09.2020
(73) Patentinhaber: B. Braun Melsungen AG, 34212 Melsungen (DE)
(72) Erfinder: Fuchs, Jürgen, 34308 Bad Emstal (DE); Hatzky, Mario, 34128 Kassel (DE)
(74) Vertreter: Patentanwälte Ruff, Wilhelm, Beier, Dauster & Partner mbB

(56) Entgegenhaltungen:
- EP-A1- 3 062 868
- EP-A1- 3 421 077
- EP-B1- 3 062 868
- US-A1- 2004 172 006

## Beschreibung

Die Erfindung betrifft einen medizinischen Fluidkonnektor für ein medizinisches Fluidleitungssystem, aufweisend einen ersten Körper mit einem in Axialrichtung erstreckten Rohrabschnitt, der einen Fluidkanal mit einem Einlass ausbildet, und mit einem koaxial zu dem Rohrabschnitt orientierten ersten Gewinde, und einen zweiten Körper mit einem in Axialrichtung erstreckten Lumen, das den Rohrabschnitt in Umfangsrichtung umgreift und einen dem Einlass zugeordneten Auslass aufweist, und mit einem zweiten Gewinde, das mit dem ersten Gewinde des ersten Körpers schraubbeweglich zusammenwirkt, wobei der Fluidkonnektor mittels einer relativen Schraubbewegung zwischen dem ersten Körper und dem zweiten Körper überführbar ist zwischen einer Schließstellung, in welcher das Lumen und der Rohrabschnitt unter Ausbildung einer den Auslass fluiddicht abdichtenden Dichtverbindung zusammenwirken, und einer Öffnungsstellung, in welcher die Dichtverbindung aufgehoben und der Auslass fluidleitend mit dem Einlass verbunden ist, und wobei der zweite Körper ein drittes Gewinde aufweist, das zu dem ersten Gewinde und zu dem zweiten Gewinde koaxial orientiert und gegenläufig gestaltet ist.

Ein derartiger medizinischer Fluidkonnektor ist aus der EP 3 421 077 A1 bekannt und zur fluidleitenden Verbindung mit einem komplementären medizinischen Fluidkonnektor für ein medizinisches Fluidleitungssystem vorgesehen. Der bekannte Fluidkonnektor weist einen ersten Körper mit einem Rohrabschnitt auf, der einen Fluidkanal mit einem Einlass ausbildet. Zudem weist der bekannte Fluidkonnektor einen zweiten Körper mit einem Lumen auf, das den Rohrabschnitt in Umfangsrichtung umgreift und mit einem dem Einlass zugeordneten Auslass versehen ist. Sowohl der erste Körper als auch der zweite Körper sind jeweils mit einem Gewinde versehen. Die beiden Gewinde wirken schraubbeweglich zusammen, wobei der Fluidkonnektor mittels einer entsprechenden Schraubbewegung zwischen einer Schließstellung und einer Öffnungsstellung überführbar ist. In der Schließstellung wirken das Lumen und der Rohrabschnitt unter Ausbildung einer Dichtverbindung fluiddicht zusammen. Die Dichtverbindung dichtet den Auslass fluiddicht ab. In der Öffnungsstellung ist diese Dichtverbindung demgegenüber aufgehoben und der Auslass freigegeben. Zudem weist der bekannte Fluidkonnektor ein an dem zweiten Körper ausgebildetes drittes Gewinde auf. Das dritte Gewinde ist koaxial zu dem ersten Gewinde und zu dem zweiten Gewinde orientiert und gegenläufig zu diesem gestaltet. Das dritte Gewinde ist zur Schraubverbindung mit einem komplementären Gewinde des besagten komplementären Fluidkonnektors vorgesehen. Zum Entlüften des bekannten Fluidkonnektors - das auch als Priming bezeichnet werden kann - weist der zweite Körper eine gesonderte Entlüftungsöffnung auf, in welche eine poröse Membran eingepasst ist. Dabei erfolgt die Entlüftung des bekannten Fluidkonnektors in der Schließstellung. Hierzu ist die Entlüftungsöffnung in der Schließstellung fluidleitend mit dem Einlass verbunden. Die Entlüftungsöffnung ist in der Öffnungsstellung gegenüber dem Einlass und dem Auslass fluiddicht abgedichtet, wozu eine gesonderte Dichtverbindung zwischen dem ersten Körper und dem zweiten Körper ausgebildet ist.

Aufgabe der Erfindung ist es, einen medizinischen Fluidkonnektor der eingangs genannten Art bereitzustellen, der eine zuverlässige Abdichtung des Auslasses in der Schließstellung sowie ein einfaches Entlüften ermöglicht und gleichzeitig einen möglichst einfachen Aufbau aufweist.

Diese Aufgabe wird dadurch gelöst, dass eine Verschlusskappe mit einem vierten Gewinde vorgesehen ist, das - in einem Auslieferungszustand des Fluidkonnektors - mit dem dritten Gewinde des zweiten Körpers lösbar verschraubt ist, wobei der Fluidkonnektor die Öffnungsstellung einnimmt, und wobei der Auslass mittels der Verschlusskappe luftdurchlässig und flüssigkeitsdicht verschlossen ist. Durch die erfindungsgemäße Lösung kann auf eine speziell im Hinblick auf das Entlüften vorgesehene Gestaltung des ersten Körpers und/oder des zweiten Körpers verzichtet werden. Insbesondere sind keine speziell zum Entlüften vorgesehenen Entlüftungsöffnungen oder Dichtverbindungen an dem ersten Körper und/oder dem zweiten Körper notwendig. Stattdessen ist erfindungsgemäß die Verschlusskappe vorgesehen, die im Auslieferungszustand lösbar auf den zweiten Körper aufgeschraubt ist. Dabei gestattet die Verschlusskappe ein einfaches und zuverlässiges Entlüften des Fluidkonnektors. Zu diesem Zweck ist die Verschlusskappe luftdurchlässig und flüssigkeitsdicht gestaltet. Durch die erfindungsgemäße Lösung ist es zudem möglich, dass der Fluidkonnektor im Auslieferungszustand die Öffnungsstellung einnimmt. Dabei ist der Auslass gleichwohl zuverlässig mittels der Verschlusskappe verschlossen, so dass eine Kontaminierung des Auslasses mit Keimen und/oder ein Eindringen von Fremdkörpern in den Auslass vermieden wird. Gleichzeitig werden gegenüber einem Auslieferungszustand, in dem der Fluidkonnektor die Schließstellung einnimmt, Setzerscheinungen an der Dichtverbindung zwischen dem Rohrabschnitt und dem Lumen vermieden. Solche Setzerscheinungen können auftreten, wenn die Dichtverbindung über einen längeren Zeitraum aufrechterhalten und somit entsprechenden Dichtkräften ausgesetzt ist. Hierdurch kann es beim späteren Gebrauch des Fluidkonnektors zu einer unerwünschten Undichtigkeit in der Schließstellung kommen. Im Ergebnis wird durch die erfindungsgemäße Lösung eine zuverlässige Abdichtung des Auslasses in der Schließstellung sowie ein einfaches Entlüften ermöglicht. Gleichzeitig weist der erfindungsgemäße Fluidkonnektor einen vergleichsweise einfachen Aufbau auf. Der Einlass ist vorzugsweise zur fluidleitenden Verbindung mit einem Schlauchabschnitt des medizinischen Fluidleitungssystems vorgesehen. Zu diesem Zweck kann der erste Körper einen Schlauchstutzen aufweisen, der zur fluidleitenden Verbindung mit dem Schlauchabschnitt eingerichtet ist. Der Auslass ist vorzugsweise zur fluidleitenden Verbindung mit einem komplementären medizinischen Fluidkonnektor vorgesehen. Zu diesem Zweck kann der zweite Körper insbesondere einen männlichen oder einen weiblichen Luer-Anschluss aufweisen. Das erste Gewinde kann in Form eines Innengewindes und das zweite Gewinde kann in Form eines hierzu komplementären Außengewindes ausgebildet sein oder umgekehrt. Bei der relativen Schraubbewegung zwischen dem ersten Gewinde und dem zweiten Gewinde bzw. zwischen dem ersten Körper und dem zweiten Körper werden diese in Umfangsrichtung relativ zueinander rotiert und in Axialrichtung relativ zueinander translatorisch verlagert. Das dritte Gewinde kann in Form eines Innengewindes und das vierte Gewinde kann in Form eines hierzu komplementären Außengewindes ausgebildet sein oder umgekehrt. Das dritte Gewinde ist dem Auslass zugeordnet. Sofern der zweite Körper einen Luer-Anschluss aufweist, ist das dritte Gewinde vorzugsweise in Form eines dem Luer-Anschluss zugeordneten Luer-Gewindes ausgebildet. Zum luftdurchlässigen und flüssigkeitsdichten Verschließen des Auslasses kann die Verschlusskappe insbesondere ein semipermeables Funktionselement oder einen semipermeablen Funktionsabschnitt aufweisen. Beispielsweise kann die Verschlusskappe ein semipermeables Filterelement oder eine semipermeable Membran aufweisen. Sofern der zweite Körper einen Luer-Anschluss aufweist, ist es vorteilhaft, wenn die Verschlusskappe einen hierzu komplementären Luer-Anschluss aufweist. In diesem Fall kann das vierte Gewinde als ein dem komplementären Luer-Anschluss zugeordnetes Luer-Gewinde ausgebildet sein. Die Erfindung ist in den Ansprüchen definiert.

In Ausgestaltung der Erfindung weist die Verschlusskappe eine semipermeable Membran auf, mittels derer der Auslass im Auslieferungszustand luftdurchlässig und flüssigkeitsdicht verschlossen ist. Diese Ausgestaltung der Erfindung ermöglicht einen besonders einfachen Aufbau des Fluidkonnektors und eine zuverlässige Funktionsweise beim Entlüften. In der Öffnungsstellung ist die semipermeable Membran fluidleitend mit dem Auslass verbunden, so dass in dem Fluidkonnektor befindliche Luft über den Fluidkanal in den Auslass und von dort bis zu der semipermeablen Membran gelangen kann. Aufgrund der semipermeablen Gestaltung kann die Luft den Fluidkonnektor durch die Membran hindurch verlassen, wobei Flüssigkeit aufgrund der flüssigkeitsdichten Gestaltung der Membran zuverlässig in dem Fluidkonnektor zurückgehalten ist.

In weiterer Ausgestaltung der Erfindung ist der Fluidkonnektor - ausgehend vom Auslieferungszustand - mittels Abschraubens der Verschlusskappe von dem zweiten Körper in die Schließstellung überführbar. Durch diese Ausgestaltung der Erfindung kann auf ein gesondertes Verschließen oder Abklemmen des Fluidkonnektors nach dem Entlüften verzichtet werden. Stattdessen ist es ausreichend, wenn die Verschlusskappe nach dem Entlüften von dem zweiten Körper abgeschraubt wird. Infolge der Schraubbewegung zwischen der Verschlusskappe und dem zweiten Körper und/oder dem ersten Körper ist der Fluidkonnektor somit gleichsam automatisch von der Öffnungsstellung in die Schließstellung überführbar. Hierzu ist es besonders vorteilhaft, wenn das erste und das zweite Gewinde sowie das dritte und das vierte Gewinde jeweils derart aufeinander abgestimmt sind, dass beim Abschrauben der Verschlusskappe zunächst das erste und das zweite Gewinde gegeneinander verschraubt werden und hiernach das dritte und das vierte Gewinde gegeneinander verschraubt werden. Zu diesem Zweck kann eine Gewindereibung zwischen dem ersten Gewinde und dem zweiten Gewinde geringer bemessen sein als eine Gewindereibung zwischen dem dritten Gewinde und dem vierten Gewinde. Dies kann insbesondere mittels einer entsprechenden Werkstoffwahl für die jeweiligen Gewinde und/oder mittels einer entsprechenden geometrischen Gestaltung erreicht sein. Alternativ oder zusätzlich kann eine Gewindeart des ersten Gewindes und des zweiten Gewindes unterschiedlich sein von einer Gewindeart des dritten Gewindes und des vierten Gewindes.

In weiterer Ausgestaltung der Erfindung weist der erste Körper einen in Axialrichtung erstreckten ersten Kragenabschnitt auf, der den Rohrabschnitt in Umfangsrichtung wenigstens abschnittsweise umgibt. Diese Ausgestaltung der Erfindung ermöglicht einen nochmals vereinfachten Aufbau des Fluidkonnektors. In radialer Richtung zwischen dem Kragenabschnitt und dem Rohrabschnitt ist vorzugsweise eine Aufnahmeaussparung ausgebildet, in welcher der zweite Körper wenigstens abschnittsweise aufgenommen sein kann. Der Kragenabschnitt kann einen in Radialrichtung außenliegenden Handhabungsabschnitt aufweisen, der einer vereinfachten manuellen Schraubbewegung zwischen dem ersten Körper und dem zweiten Körper dient. Zudem kann das erste Gewinde an dem Kragenabschnitt ausgebildet sein.

In weiterer Ausgestaltung der Erfindung ist das erste Gewinde in Form eines Innengewindes an dem ersten Kragenabschnitt ausgebildet. Hierdurch kann ein nochmals vereinfachter Aufbau des Fluidkonnektors erreicht werden. Das Innengewinde ist vorzugsweise an einem in Radialrichtung innenliegenden Wandabschnitt des ersten Kragenabschnitts ausgebildet.

In weiterer Ausgestaltung der Erfindung weist der Rohrabschnitt einen in Axialrichtung elastisch federbeweglichen Federabschnitt auf, mittels dessen - in der Schließstellung - eine elastische Vorspannung der Dichtverbindung bewirkt ist. Durch die elastische Vorspannung der Dichtverbindung kann eine verbesserte Dichtigkeit in der Schließstellung erreicht werden. Die elastisch federbewegliche Gestaltung des Federabschnitts kann mittels einer entsprechenden Gestaltgebung und/oder Werkstoffwahl erreicht sein.

In weiterer Ausgestaltung der Erfindung ist der Federabschnitt in Form einer Schraubenfeder ausgebildet. Diese Ausgestaltung der Erfindung erlaubt einen besonders einfachen Aufbau des Fluidkonnektors.

In weiterer Ausgestaltung der Erfindung weist der zweite Körper einen männlichen Luer-Konus auf, der dem Auslass zugeordnet ist und durch welchen das Lumen erstreckt ist. Demnach ist der Fluidkonnektor bei dieser Ausgestaltung der Erfindung in Form eines männlichen Luer-Konnektors ausgebildet und zur fluidleitenden Verbindung mit einem komplementären weiblichen Luer-Konnektor vorgesehen. Hierbei ist es besonders vorteilhaft, wenn die Verschlusskappe einen weiblichen Luer-Konus aufweist, der im Auslieferungszustand fluiddicht mit dem männlichen Luer-Konus des zweiten Körpers zusammengefügt ist.

In weiterer Ausgestaltung der Erfindung weist der zweite Körper einen in Axialrichtung erstreckten zweiten Kragenabschnitt auf, der den männlichen Luer-Konus in Umfangsrichtung wenigstens abschnittsweise umgreift und an welchem das dritte Gewinde in Form eines Innengewindes ausgebildet ist. Bei dieser Ausgestaltung der Erfindung ist das dritte Gewinde demnach ein Luer-Gewinde, das dem männlichen Luer-Konus des zweiten Körpers zugeordnet ist. Das vierte Gewinde ist bei dieser Ausgestaltung der Erfindung demnach in Form eines Außengewindes ausgebildet, das komplementär zu dem in Form eines Innengewindes ausgebildeten dritten Gewinde gestaltet ist.

Weitere Vorteile und Merkmale der Erfindung ergeben sich aus den Ansprüchen sowie aus der nachfolgenden Beschreibung eines bevorzugten Ausführungsbeispiels der Erfindung, das anhand der Zeichnungen dargestellt ist.
- Fig. 1: zeigt in einer schematischen Seitenansicht eine Ausführungsform eines erfindungsgemäßen medizinischen Fluidkonnektors, wobei der Fluidkonnektor einen Auslieferungszustand einnimmt und einends eine Verschlusskappe aufweist,
- Fig. 2: in schematischer Längsschnittdarstellung den medizinischen Fluidkonnektor nach Fig. 1 im Auslieferungszustand, wobei der Fluidkonnektor gleichzeitig eine Öffnungsstellung einnimmt und
- Fig. 3: in einer weiteren schematischen Längsschnittdarstellung den Fluidkonnektor nach den Fig. 1 und 2, wobei die Verschlusskappe abgeschraubt und der Fluidkonnektor in eine Schließstellung überführt ist.

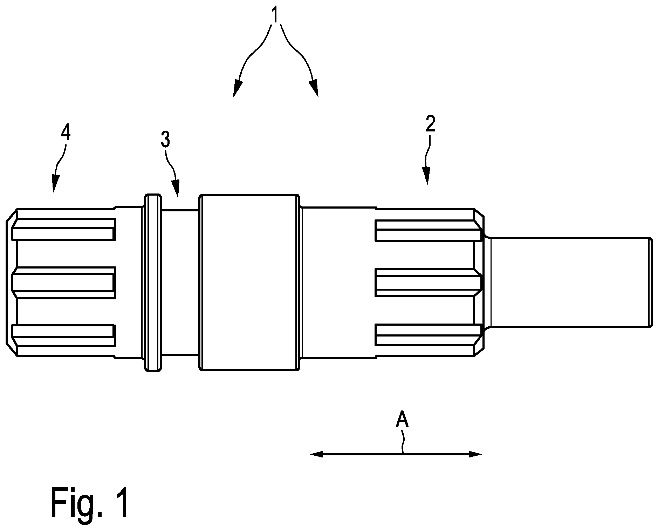

Gemäß den Fig. 1 bis 3 ist ein medizinischer Fluidkonnektor für ein zeichnerisch nicht näher dargestelltes medizinisches Fluidleitungssystem vorgesehen. Bei dem medizinischen Fluidleitungssystem handelt es sich vorliegend um ein IV-Set, das auch als Infusionsset oder Überleitungssystem bezeichnet werden kann.

Der medizinische Fluidkonnektor 1 weist einen ersten Körper 2, einen zweiten Körper 3 sowie eine Verschlusskappe 4 auf.

Der erste Körper 2 weist einen in Axialrichtung A erstreckten Rohrabschnitt 5 auf, der einen Fluidkanal 6 mit einem Einlass 7 ausbildet. Der erste Körper 2 weist zudem ein erstes Gewinde 8 auf, das koaxial zu dem Rohrabschnitt 5 orientiert ist.

Der zweite Körper 3 weist ein in Axialrichtung A erstrecktes Lumen 9 auf. Das Lumen 9 umgreift den Rohrabschnitt 5 in Umfangsrichtung und weist einen dem Einlass 7 zugeordneten Auslass 10 auf. Zudem weist der zweite Körper 3 ein zweites Gewinde 11 auf, das mit dem ersten Gewinde 8 des ersten Körpers 2 schraubbeweglich zusammenwirkt.

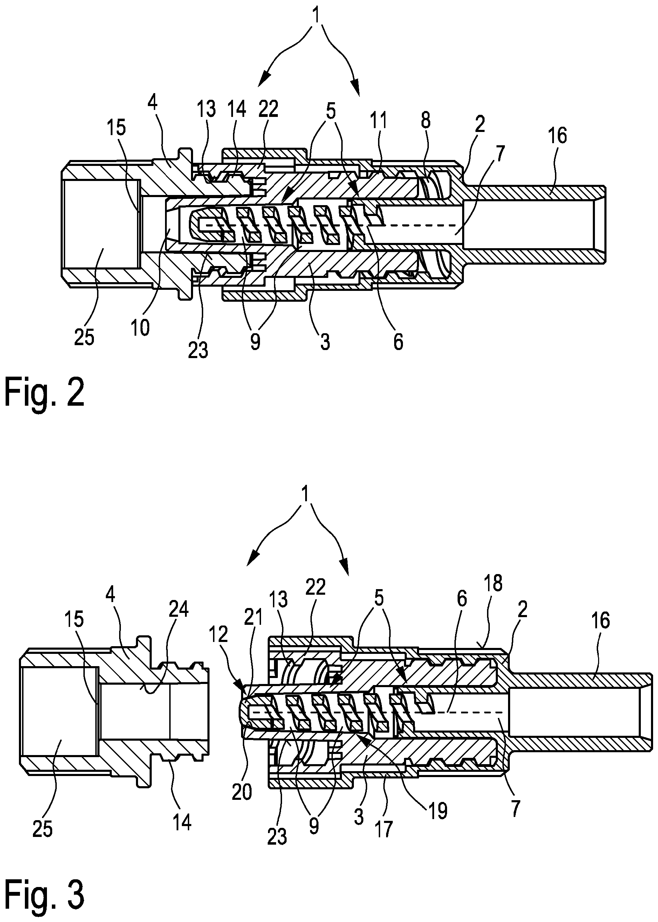

Der Fluidkonnektor 1 ist mittels einer relativen Schraubbewegung zwischen dem ersten Gewinde 8 und dem zweiten Gewinde 11 bzw. zwischen dem ersten Körper 2 und dem zweiten Körper 3 zwischen einer Öffnungsstellung (Fig. 2) und einer Schließstellung überführbar (Fig. 3). In der Schließstellung wirken das Lumen 9 und der Rohrabschnitt 5 unter Ausbildung einer den Auslass 10 fluiddicht abdichtenden Dichtverbindung 12 zusammen. Demgegenüber ist der Auslass 10 in der Öffnungsstellung freigegeben und fluidleitend mit dem Einlass 7 verbunden, wozu die Dichtverbindung 12 zwischen dem Lumen 9 und dem Rohrabschnitt 5 aufgehoben ist.

Der zweite Körper 3 weist zudem ein drittes Gewinde 13 auf, das zu dem ersten Gewinde 8 und zu dem zweiten Gewinde 11 koaxial orientiert ist. Zudem ist das dritte Gewinde 13 gegenläufig zu dem ersten Gewinde 8 und zu dem zweiten Gewinde 11 gestaltet. Vorliegend sind das erste Gewinde 8 und das zweite Gewinde 11 jeweils als Linksgewinde gestaltet, wohingegen das dritte Gewinde 13 vorliegend als ein hierzu gegenläufiges Rechtsgewinde gestaltet ist. Dies ist jedoch nicht zwingend. Bei einer zeichnerisch nicht dargestellten Ausführungsform kann das dritte Gewinde 13 als Linksgewinde gestaltet sein, wobei das erste Gewinde 8 und das zweite Gewinde 11 dementsprechend als Rechtsgewinde gestaltet sein können.

In dem anhand der Fig. 1 und 2 ersichtlichen Zustand - der auch als Auslieferungszustand bezeichnet werden kann - ist die Verschlusskappe 4 lösbar mit dem zweiten Körper 3 verschraubt. Hierzu weist die Verschlusskappe 4 ein viertes Gewinde 14 auf, das komplementär zu dem dritten Gewinde 13 gestaltet ist. Der Fluidkonnektor 1 nimmt im Auslieferungszustand (Fig. 1, 2) die Öffnungsstellung ein, wobei der Auslass 10 mittels der Verschlusskappe 4 luftdurchlässig und flüssigkeitsdicht verschlossen ist.

Zum luftdurchlässigen und flüssigkeitsdichten Verschließen des Auslasses 10 weist die Verschlusskappe 4 vorliegend eine semipermeable Membran 15 auf, was jedoch nicht zwingend ist. Anstelle der Membran 15 kann bei einer nicht dargestellten Ausführungsform insbesondere ein poröser Filterabschnitt oder ein poröses Filterelement vorgesehen sein.

Nachfolgend wird die Funktionsweise des medizinischen Fluidkonnektors 1 beim Entlüften des Fluidleitungssystems näher beschrieben. Ein solches Entlüften kann auch als Priming bezeichnet werden.

Zum Entlüften wird der Fluidkonnektor 1 in dem anhand der Fig. 1 und 2 ersichtlichen Auslieferungszustand ausgehend vom Einlass 7 in Richtung des Auslasses 10 mit einer nicht näher bezeichneten medizinischen Flüssigkeit befüllt. Hierzu wird der Fluidkonnektor 1 einlassseitig auf noch näher beschriebene Weise mit einem Schlauchabschnitt des Fluidleitungssystems fluidleitend verbunden sein, der seinerseits wiederum mit einem die medizinische Flüssigkeit beinhaltenden Infusionsbehälter verbunden sein kann. Dabei gelangt die medizinische Flüssigkeit durch den Einlass 7 in den Rohrabschnitt 5 und damit in den Fluidkanal 6. Da der Fluidkonnektor 1 im Auslieferungszustand die Öffnungsstellung einnimmt, gelangt die medizinische Flüssigkeit ausgehend von dem Fluidkanal 6 weiter durch den Auslass 10 bis zu der semipermeablen Membran 15. Dabei wird die medizinische Flüssigkeit mittels der semipermeablen Membran 15 zurückgehalten, wohingegen etwaige Luftblasen durch die semipermeable Membran 15 aus dem Fluidkonnektor 1 - und dem daran angeschlossenen Fluidleitungssystem - ausgespült werden können.

Nach dem auf diese Weise erfolgten Entlüften des Fluidkonnektors 1 kann die Verschlusskappe 4 schraubbeweglich von dem zweiten Körper 3 entfernt werden. Aufgrund der vorliegenden Gestaltung des dritten Gewindes 13 und des vierten Gewindes 14 wird die Verschlusskappe 4 hierzu vorliegend entgegen dem Uhrzeigersinn relativ zu dem ersten Körper 2 und dem zweiten Körper 3 in Umfangsrichtung rotiert. Dies bewirkt zudem, dass das erste Gewinde 8 und das zweite Gewinde 11 gegeneinander verschraubt werden, wodurch der zweite Körper 3 in Axialrichtung A relativ zu dem ersten Körper 2 verlagert wird. Aufgrund dieser Verlagerung wird der Fluidkonnektor 1 ausgehend von der Öffnungsstellung in die Schließstellung überführt (Fig. 3) und die Dichtverbindung 12 zwischen dem Lumen 9 und dem Rohrabschnitt 5 ausgebildet. Wird die Verschlusskappe 4 hiernach weiter entgegen dem Uhrzeigersinn rotiert, bewirkt dies, dass die Schraubverbindung zwischen dem dritten Gewinde 13 und dem vierten Gewinde 14 gelöst wird. Hiernach nimmt der Fluidkonnektor 1 die anhand Fig. 3 ersichtliche Konfiguration ein, in der die Verschlusskappe 4 von dem zweiten Körper 3 gelöst und die Dichtverbindung 12 ausgebildet bzw. die Schließstellung eingenommen ist.

Nachfolgend werden weitere funktionelle und körperliche Merkmale der vorliegenden Ausführungsform näher erläutert, wobei die damit einhergehende Gestaltung des Fluidkonnektors 1 als rein exemplarisch zu verstehen ist.

Der erste Körper 2 weist vorliegend einen Schlauchstutzen 16 auf, der zur fluidleitenden Verbindung mit einem zeichnerisch nicht dargestellten Schlauchabschnitt des Fluidleitungssystems eingerichtet ist. Zur fluidleitenden Verbindung wird der Schlauchabschnitt in Axialrichtung A in den Schlauchstutzen 16 eingesteckt. Der Schlauchstutzen 16 mündet einends in den Einlass 7 des Fluidkanals 6.

Weiterhin weist der erste Körper 2 vorliegend einen ersten Kragenabschnitt 17 auf, der in Axialrichtung A erstreckt ist und den Rohrabschnitt 5 in Umfangsrichtung umgibt. Der erste Kragenabschnitt 17 ist in Radialrichtung nach außen von dem Rohrabschnitt 5 beabstandet. Hierdurch ist ein nicht näher bezeichneter Aufnahmeraum zwischen dem Rohrabschnitt 5 und dem Kragenabschnitt 17 ausgebildet, in welchem der zweite Körper 3 schraubbeweglich und in Axialrichtung A linear verlagerbar aufgenommen ist. Der Kragenabschnitt 17 weist eine in Axialrichtung A mehrfach abgestufte kreiszylindrische Außenkontur 18 auf.

Das erste Gewinde 8 ist vorliegend an dem Kragenabschnitt 17 ausgebildet und an einem dem Schlauchstutzen 16 zugewandten Endbereich des Rohrabschnitts 5 angeordnet. Das erste Gewinde ist vorliegend in Form eines Innengewindes 8 und das zweite Gewinde ist dementsprechend vorliegend in Form eines Außengewindes 11 ausgebildet, was jedoch nicht zwingend ist. Bei einer nicht dargestellten Ausführungsform kann stattdessen das erste Gewinde in Form eines Außengewindes und das zweite Gewinde in Form eines hierzu komplementären Innengewindes ausgebildet sein.

Der in Axialrichtung A längserstreckte Rohrabschnitt 5 weist vorliegend einen Federabschnitt 19 auf. Der Federabschnitt 19 ist in Axialrichtung A elastisch federbeweglich ausgestaltet. Der Federabschnitt 19 dient einer elastischen Vorspannung der Dichtverbindung 12. Dementsprechend ist der Federabschnitt 19 in der Schließstellung (Fig. 3) unter Einwirkung des zweiten Körpers 3 elastisch gestaucht. In der Öffnungsstellung (Fig. 2) ist der Federabschnitt 19 demgegenüber vollständig in Axialrichtung A expandiert.

Der Federabschnitt ist vorliegend in Form einer Schraubenfeder 19 ausgebildet, was jedoch nicht zwingend ist. Bei einer nicht dargestellten Ausführungsform kann der Federabschnitt beispielsweise in Form eines faltenbalgartigen Elements oder durch eine entsprechend dünnwandige Gestaltung des Rohrabschnitts 5 ausgebildet sein.

Das Lumen 9 des zweiten Körpers 3 umgreift den Rohrabschnitt 5 in der Schließstellung fluiddicht. Zu diesem Zweck ist eine nicht näher bezeichnete Innenkontur des Lumens 9 auf eine nicht näher bezeichnete Außenkontur des Rohrabschnitts 5 abgestimmt. Die Innenkontur des Lumens 9 weist eine im Wesentlichen kreiszylindrische Grundform auf und ist einends im Bereich der Dichtverbindung 12 in Radialrichtung verjüngt. Diese Verjüngung des Lumens 9 bildet eine Art Kegelsitz 20 aus, der in der Schließstellung fluiddicht mit einem dem Einlass 7 abgewandten Stirnende 21 des Rohrabschnitts 5 zusammenwirkt. Das Stirnende 21 weist dementsprechend eine zu dem Kegelsitz 20 komplementäre kegelige Formgebung auf. In der Öffnungsstellung sind der Kegelsitz 20 und das Stirnende 21 in Axialrichtung A relativ zueinander verlagert, so dass die Dichtverbindung 12 aufgehoben und der Auslass 10 freigegeben ist. Dabei ist der Auslass 10 in der Öffnungsstellung fluidleitend mit dem Fluidkanal 6 und dem Einlass 7 verbunden.

Weiter weist der zweite Körper 3 vorliegend einen zweiten Kragenabschnitt 22 auf. Der zweite Kragenabschnitt 22 ist in Axialrichtung A erstreckt und umgibt das Lumen 9 in Umfangsrichtung wenigstens abschnittsweise. Dabei ist der zweite Kragenabschnitt 22 an einem dem zweiten Gewinde 11 abgewandten Stirnende des zweiten Körpers 3 angeordnet. Das dritte Gewinde 13 ist an dem zweiten Kragenabschnitt 22 ausgebildet. Vorliegend ist das dritte Gewinde in Form eines Innengewindes 13 und das vierte Gewinde ist dementsprechend in Form eines hierzu komplementären Außengewindes 14 ausgebildet. Bei einer nicht dargestellten Ausführungsform kann stattdessen das dritte Gewinde in Form eines Außengewindes und das vierte Gewinde in Form eines Innengewindes ausgebildet sein.

Weiterhin weist der zweite Körper 3 vorliegend einen männlichen Luer-Konus 23 auf, der dem Auslass 10 zugeordnet ist. Der Luer-Konus 23 ist koaxial zu dem Lumen 9 und damit auch koaxial zu dem Rohrabschnitt 5 erstreckt, wobei sich das Lumen 9 in Axialrichtung A abschnittsweise durch den Luer-Konus 23 erstreckt. Der zweite Kragenabschnitt 22 umgibt den Luer-Konus 23 in Umfangsrichtung und ist koaxial zu diesem orientiert. Dabei bilden der männliche Luer-Konus 23 und das dritte Gewinde 13 einen männlichen Luer-Lock-Anschluss 23, 13.

Das vierte Gewinde ist vorliegend in Form eines Außengewindes 14 an einem dem zweiten Körper 3 zugewandten Stirnende der Verschlusskappe 4 angeordnet. Weiterhin weist die Verschlusskappe 4 vorliegend einen koaxial zu dem vierten Gewinde 14 orientierten weiblichen Luer-Konus 24 auf, der zur fluiddichten Verbindung mit dem männlichen Luer-Konus 23 vorgesehen ist. Dabei bilden das vierte Gewinde 14 und der weibliche Luer-Konus 24 einen weiblichen Luer-Lock-Anschluss 14, 24. In dem anhand der Fig. 1 und 2 ersichtlichen Auslieferungszustand sind der weibliche Luer-Lock-Anschluss 14, 24 und der männliche Luer-Lock-Anschluss 13, 23 auf grundsätzlich bekannte Weise zusammengefügt.

Die semipermeable Membran 15 ist vorliegend in Form einer Kreisscheibe ausgebildet und in einer zylindrischen Aussparung 25 der Verschlusskappe 4 angeordnet. Die zylindrische Aussparung 25 ist gegenüber dem weiblichen Luer-Konus 24 im Durchmesser vergrößert und mittels der semipermeablen Membran 15 luftdurchlässig und flüssigkeitsdicht mit demselben verbunden.

Ausgehend von dem anhand Fig. 3 ersichtlichen Zustand ist der Fluidkonnektor 1 fluidleitend mit einem zeichnerisch nicht dargestellten komplementären Fluidkonnektor verbindbar. Zur Verbindung mit dem Fluidkonnektor 1 weist der komplementäre Fluidkonnektor einen weiblichen Luer-Lock-Anschluss auf, der auf grundsätzlich bekannte Weise fluiddicht mit dem männlichen Luer-Lock-Anschluss 13, 23 des zweiten Körpers 3 verbindbar ist. Hierzu wird der komplementäre Fluidkonnektor im Uhrzeigersinn mit dem zweiten Körper 3 und dem ersten Körper 2 verschraubt. Hierbei wird zunächst die fluiddichte Verbindung zwischen dem weiblichen Luer-Lock-Anschluss des komplementären Fluidkonnektors und dem männlichen Luer-Lock-Anschluss 13, 23 des zweiten Körpers 3 hergestellt. Nach einem vollständigen Verschrauben wird der zweite Körper 3 bei einer weiteren manuellen Drehbetätigung des komplementären Fluidkonnektors relativ zu dem ersten Körper 2 rotiert. Hierdurch wirken das erste Gewinde 8 und das zweite Gewinde 11 schraubbeweglich zusammen. Aufgrund der gegenläufigen Gestaltung des ersten und zweiten Gewindes 8, 11 gegenüber dem dritten und vierten Gewinde 13, 14 wird der zweite Körper 3 hierdurch in Axialrichtung A gegenüber dem ersten Körper 2 verlagert, wodurch der Fluidkonnektor 1 in die Öffnungsstellung überführt wird.

## Patentansprüche

1. Medizinischer Fluidkonnektor (1) für ein medizinisches Fluidleitungssystem, aufweisend
- einen ersten Körper (2) mit einem in Axialrichtung (A) erstreckten Rohrabschnitt (5), der einen Fluidkanal (6) mit einem Einlass (7) ausbildet, und mit einem koaxial zu dem Rohrabschnitt (5) orientierten ersten Gewinde (8), und
- einen zweiten Körper (3) mit einem in Axialrichtung (A) erstreckten Lumen (9), das den Rohrabschnitt (5) in Umfangsrichtung umgreift und einen dem Einlass (7) zugeordneten Auslass (10) aufweist, und mit einem zweiten Gewinde (11), das mit dem ersten Gewinde (8) des ersten Körpers (2) schraubbeweglich zusammenwirkt,
- wobei der Fluidkonnektor (1) mittels einer relativen Schraubbewegung zwischen dem ersten Körper (2) und dem zweiten Körper (3) überführbar ist zwischen
- einer Schließstellung, in welcher das Lumen (9) und der Rohrabschnitt (5) unter Ausbildung einer den Auslass (10) fluiddicht abdichtenden Dichtverbindung (12) zusammenwirken, und
- einer Öffnungsstellung, in welcher die Dichtverbindung (12) aufgehoben und der Auslass (10) fluidleitend mit dem Einlass (7) verbunden ist,
- und wobei der zweite Körper (3) ein drittes Gewinde (13) aufweist, das zu dem ersten Gewinde (8) und zu dem zweiten Gewinde (11) koaxial orientiert und gegenläufig gestaltet ist,
- **dadurch gekennzeichnet, dass** eine Verschlusskappe (4) mit einem vierten Gewinde (14) vorgesehen ist, das in einem Auslieferungszustand des Fluidkonnektors (1) mit dem dritten Gewinde (13) des zweiten Körpers (3) lösbar verschraubt ist, wobei der Fluidkonnektor (1) die Öffnungsstellung einnimmt, und wobei der Auslass (10) mittels der Verschlusskappe (4) luftdurchlässig und flüssigkeitsdicht verschlossen ist.

2. Medizinischer Fluidkonnektor (1) nach Anspruch 1, **dadurch gekennzeichnet, dass** die Verschlusskappe (4) eine semipermeable Membran (15) aufweist, mittels derer der Auslass (10) im Auslieferungszustand luftdurchlässig und flüssigkeitsdicht verschlossen ist.

3. Medizinischer Fluidkonnektor (1) nach Anspruch 1 oder 2, **dadurch gekennzeichnet, dass** der Fluidkonnektor (1) - ausgehend vom Auslieferungszustand - mittels Abschraubens der Verschlusskappe (4) von dem zweiten Körper (3) in die Schließstellung überführbar ist.

4. Medizinischer Fluidkonnektor (1) nach einem der vorhergehenden Ansprüche, **dadurch gekennzeichnet, dass** der erste Körper (2) einen in Axialrichtung (A) erstreckten ersten Kragenabschnitt (17) aufweist, der den Rohrabschnitt (5) in Umfangsrichtung wenigstens abschnittsweise umgibt.

5. Medizinischer Fluidkonnektor (1) nach Anspruch 4, **dadurch gekennzeichnet, dass** das erste Gewinde in Form eines Innengewindes (8) an dem ersten Kragenabschnitt (17) ausgebildet ist.

6. Medizinischer Fluidkonnektor (1) nach einem der vorhergehenden Ansprüche, **dadurch gekennzeichnet, dass** der Rohrabschnitt (5) einen in Axialrichtung (A) elastisch federbeweglichen Federabschnitt (19) aufweist, mittels dessen - in der Schließstellung - eine elastische Vorspannung der Dichtverbindung (12) bewirkt ist.

7. Medizinischer Fluidkonnektor (1) nach Anspruch 6, **dadurch gekennzeichnet, dass** der Federabschnitt in Form einer Schraubenfeder (19) ausgebildet ist.

8. Medizinischer Fluidkonnektor (1) nach einem der vorhergehenden Ansprüche, **dadurch gekennzeichnet, dass** der zweite Körper (3) einen männlichen Luer-Konus (23) aufweist, der dem Auslass (10) zugeordnet ist und durch welchen das Lumen (9) erstreckt ist.

9. Medizinischer Fluidkonnektor (1) nach einem der vorhergehenden Ansprüche, **dadurch gekennzeichnet, dass** der zweite Körper (3) einen in Axialrichtung (A) erstreckten zweiten Kragenabschnitt (22) aufweist, der den männlichen Luer-Konus (23) in Umfangsrichtung wenigstens abschnittsweise umgreift und an welchem das dritte Gewinde in Form eines Innengewindes (13) ausgebildet ist.

## Claims

1. Medical fluid connector (1) for a medical fluid line system, having
a first body (2) with a tube section (5) which extends in an axial direction (A) and which forms a fluid channel (6) with an inlet (7), and with a first thread (8), which is oriented so as to be coaxial with the tube section (5), and
a second body (3) with a lumen (9) which extends in axial direction (A) and which engages around the tube section (5) in a circumferential direction and which has an outlet (10) which is associated with the inlet (7), and with a second thread (11), which interacts with the first thread (8) of the first body (2) in a manner movable by screwing,
wherein the fluid connector (1) is, by means of a relative screwing movement between the first body (2) and the second body (3), able to be transferred between
a closed position, in which the lumen (9) and the tube section (5) interact so as to form a sealing connection (12) which, in a fluid-tight manner, seals off the outlet (10), and
an open position, in which the sealing connection (12) is eliminated and the outlet (10) is connected in a fluid-conducting manner to the inlet (7),
and wherein the second body (3) has a third thread (13), which is oriented so as to be coaxial with the first thread (8) and with the second thread (11) and is designed to rotate in the opposite direction in relation to said first and second threads,
**characterized in that** provision is made of a closure cap (4) with a fourth thread (14) which in a delivery state of the fluid connector (1) is detachably screwed to the third thread (13) of the second body (3), wherein the fluid connector (1) assumes the open position, and wherein the outlet (10) is closed off in an air-permeable and liquid-tight manner by means of the closure cap (4).

2. Medical fluid connector (1) according to Claim 1, **characterized in that** the closure cap (4) has a semi-permeable membrane (15) by means of which the outlet (10), in the delivery state, is closed off in an air-permeable and liquid-tight manner.

3. Medical fluid connector (1) according to Claim 1 or 2, **characterized in that** the fluid connector (1) - proceeding from the delivery state - is able to be transferred into the closed position by means of unscrewing of the closure cap (4) from the second body (3).

4. Medical fluid connector (1) according to one of the preceding claims, **characterized in that** the first body (2) has a first collar section (17), which extends in an axial direction (A) and at least sectionally surrounds the tube section (5) in a circumferential direction.

5. Medical fluid connector (1) according to Claim 4, **characterized in that** the first thread is formed in the form of an inner thread (8) on the first collar section (17).

6. Medical fluid connector (1) according to one of the preceding claims, **characterized in that** the tube section (5) has a spring section (19) which is elastically resiliently movable in an axial direction (A) and by means of which - in the closed position - elastic preloading of the sealing connection (12) is brought about.

7. Medical fluid connector (1) according to Claim 6, **characterized in that** the spring section is in the form of a helical spring (19).

8. Medical fluid connector (1) according to one of the preceding claims, **characterized in that** the second body (3) has a male Luer cone (23), which is associated with the outlet (10) and through which the lumen (9) extends.

9. Medical fluid connector (1) according to one of the preceding claims, **characterized in that** the second body (3) has a second collar section (22), which extends in axial direction (A) and at least sectionally engages around the male Luer cone (23) in a circumferential direction and on which the third thread is formed in the form of an inner thread (13) .

## Revendications

1. Connecteur de fluide médical (1) pour un système de conduite de fluide médical, comprenant
un premier corps (2) comprenant une section de tube (5) s'étendant dans une direction axiale (A), qui forme un canal de fluide (6) avec une entrée (7), et un premier filetage (8) orienté coaxialement à la section de tube (5), et
un deuxième corps (3) avec une lumière (9) s'étendant dans la direction axiale (A), qui entoure la section de tube (5) dans la direction circonférentielle et qui présente à l'entrée (7), et avec un deuxième filetage (11) qui coopère par vissage avec le premier filetage (8) du premier corps (2),
le connecteur de fluide (1) pouvant être transféré au moyen d'un mouvement de vissage relatif entre le premier corps (2) et le deuxième corps (3) entre
une position de fermeture dans laquelle la lumière (9) et le tronçon de tube (5) coopèrent en formant une liaison étanche (12) qui rend la sortie (10) étanche aux fluides, et
une position d'ouverture, dans laquelle la liaison étanche (12) est supprimée et la sortie (10) est reliée à l'entrée (7) de manière à conduire le fluide,
et dans lequel le deuxième corps (3) présente un troisième filetage (13) qui est orienté coaxialement par rapport au premier filetage (8) et au deuxième filetage (11) et qui est conçu pour tourner en sens inverse,
**caractérisé en ce qu'**il est prévu un capuchon de fermeture (4) avec un quatrième filetage (14) qui, dans un état de livraison du connecteur de fluide (1), est vissé de manière amovible avec le troisième filetage (13) du deuxième corps (3), le connecteur de fluide (1) prenant la position d'ouverture, et la sortie (10) étant fermée de manière perméable à l'air et étanche aux liquides au moyen du capuchon de fermeture (4).

2. Connecteur de fluide médical (1) selon la revendication 1, **caractérisé en ce que** le capuchon de fermeture (4) présente une membrane semi-perméable (15) au moyen de laquelle la sortie (10) est fermée de manière perméable à l'air et étanche aux liquides dans l'état de livraison.

3. Connecteur fluidique médical (1) selon la revendication 1 ou 2, **caractérisé en ce que en ce que** le connecteur de fluide (1) - en partant de l'état de livraison - peut être transféré dans la position de fermeture en dévissant le capuchon de fermeture (4) du deuxième corps (3).

4. Connecteur de fluide médical (1) selon l'une des revendications précédentes, **caractérisé en ce que** le premier corps (2) présente une première section de collerette (17) s'étendant dans la direction axiale (A), qui entoure au moins partiellement la section de tube (5) dans la direction circonférentielle.

5. Connecteur de fluide médical (1) selon la revendication 4, **caractérisé en ce que** le premier filetage est formé sous la forme d'un filetage interne (8) sur la première partie de collerette (17).

6. Connecteur de fluide médical (1) selon l'une des revendications précédentes, **caractérisé en ce que** le tronçon de tube (5) présente un tronçon de ressort (19) mobile élastiquement dans la direction axiale (A), au moyen duquel - en position de fermeture - une précontrainte élastique de la liaison d'étanchéité (12) est provoquée.

7. Connecteur de fluide médical (1) selon la revendication 6, **caractérisé en ce que** la section de ressort est réalisée sous la forme d'un ressort hélicoidal (19).

8. Connecteur de fluide médical (1) selon l'une quelconque des revendications précédentes, **caractérisé en ce que** le deuxième corps (3) comprend un cône Luer mâle (23) associé à la sortie (10) et à travers lequel s'étend la lumière (9).

9. Connecteur de fluide médical (1) selon l'une des revendications précédentes, **caractérisé en ce que** le deuxième corps (3) présente une deuxième section de collerette (22) s'étendant dans la direction axiale (A), qui entoure le cône Luer mâle (23) au moins par sections dans la direction périphérique et sur laquelle le troisième filetage est réalisé sous la forme d'un filetage intérieur (13).
